# EUROPÄISCHE PATENTSCHRIFT

(11) **EP 3 520 743 B1**
(45) Veröffentlichungstag und Bekanntmachung des Hinweises auf die Patenterteilung: **08.04.2020**
(21) Anmeldenummer: 18155135.9
(22) Anmeldetag: 05.02.2018
(51) Int. Cl.: A61F 5/56

(54) **VORRICHTUNG ZUR SCHNARCHTHERAPIE UND VERFAHREN ZUR HERSTELLUNG**
DEVICE FOR THERAPY AGAINST SNORING AND METHOD FOR PRODUCING
DISPOSITIF POUR THÉRAPIE CONTRE LE RONFLEMENT ET PROCÉDÉ DE FORMATION

(43) Veröffentlichungstag der Anmeldung: 07.08.2019
(73) Patentinhaber: Stavrou, Petros, 22453 Hamburg (DE)
(72) Erfinder:
(74) Vertreter: RGTH

(56) Entgegenhaltungen:
- WO-A1-2016/078836
- WO-A2-2007/020197
- US-A- 5 915 385
- US-A1- 2016 022 473

## Beschreibung

Die vorliegende Erfindung befasst sich mit einer Vorrichtung zur Schnarchtherapie umfassend einen Befestigungsabschnitt zur Befestigung an Zähnen eines Oberkiefers und einen Funktionsabschnitt, wobei der Funktionsabschnitt im am Oberkiefer angeordneten Zustand in einer dorsalen Richtung über eine A-Linie hinausragt und an einem weichen Gaumen in Ruhelage anliegt, wobei der Funktionsabschnitt ausgebildet ist, bei einem Schluckvorgang eine Gegenkraft auf am Schluckvorgang beteiligte Muskeln, insbesondere auf den Musculus tensor veli palatini und den Musculus levator veli palatini, auszuüben. Ferner betrifft die vorliegende Erfindung ein Verfahren zur Herstellung einer Vorrichtung zur Schnarchtherapie. Die Verwendung einer Vorrichtung zur Schnarchtherapie wird beschrieben.

### Technologischer Hintergrund

Schlafbezogene Atemstörungen wie das Schnarchen betreffen einen wesentlichen Teil der Bevölkerung. Als Ursache des Schnarchens wird unter anderem auch ein zu geringer Tonus der Muskulatur des Gaumens und der Rachenwege angesehen. Im Schlaf führt der zu geringe Tonus zu einem Erschlaffen der Muskulatur, und es kommt zu Vibrationen des Gaumensegels. Als Folgen des Schnarchens können das Schlaf-Apnoe-Hyponoe-Syndrom (SAHS) oder das Obstruktive Schlaf-Apnoe-Syndrom (OSAS) auftreten, bei dem es zu Atemaussetzern während des Schlafes kommt. Schnarchen und die mit dem Schnarchen verbundenen Folgen führen zu Einschränkungen des täglichen Lebens der Betroffenen. Häufig tritt zum Beispiel Tagesmüdigkeit auf. Auch das Risiko von Folgeerkrankungen wie Herzkreislauferkrankungen wird erhöht.

Zur Verhinderung oder zur Reduzierung des nächtlichen Schnarchens sind aus dem Stand der Technik Vorrichtungen zur Schnarchverhinderung bekannt, welche zur Nacht in den Mundraum des Betroffenen eingesetzt werden. Bügel an den bekannten Vorrichtungen halten die Zunge, um zu verhindern, dass diese dorsal in Richtung des Rachenraums fällt und eine Blockade der Atemwege verursacht.

Die WO 2016/078836 A1 stellt den nächstliegenden Stand der Technik dar und offenbart ein Rachen- und Gaumenmuskelaufbaugerät, wobei das Gerät als Schiene zur Befestigung am Oberkieferzahnkranz ausgebildet ist und das ferner ausgebildet ist, in Ruhelage die Gaumenbögen im Bereich der Plica semilunaris zu tangieren und beim Schluckakt die Plica semilunaris unter mechanischer Behinderung der Gaumenbögen zu berühren.

Aus der DE 20 2014 001 272 ist eine intraorale Applikation zur Behandlung schlafbezogener Atmungsstörungen bekannt, wobei die Applikation einen Bügel aufweist, wobei der Bügel in den Bereich hinter das Gaumenzäpfchen und wieder zurück an die Vorrichtung geführt und von dieser gehalten wird.

Die US 2016/0287429 A1 offenbart eine Schnarchverhinderungsvorrichtung, welche ein Luftstromblockiermittel aufweist. Das Luftstromblockiermittel unterbricht den Luftstrom durch die Mund- und Rachenhöhle, sodass der Träger gezwungen wird, durch die Nase zu atmen. Ferner hält das Luftstromblockiermittel die Zunge fest, sodass diese nicht in den Rachen fallen kann.

Aus der EP 2 380 533 A1 ist eine auf die Zähne eines Benutzers aufsetzbare Vorrichtung bekannt, welche einen Bügel aufweist. Im aufgesetzten Zustand weist die Vorrichtung einen nach unten und zum Rachen gerichteten Bügel auf, mit welchem die Zunge beim Schlaf in einer Position gehalten wird, sodass diese nicht rückwärts in den Rachen fallen kann.

Die US 5,915,385 offenbart eine Anti-Schnarchvorrichtung, bei der ein zapfenartiger und nach unten gerichteter Vorsprung Druck auf den weichen Gaumen ausübt, um das Herabsacken des Gaumensegels während des Schlafes zu verhindern. Die Anti-Schnarchvorrichtung wird während der Schlafphase getragen.

In der WO 2007/020197 A2 ist eine Vorrichtung zur Behandlung von Schnarchgeräuschen, Atemunterbrüchen und obstruktiver Schlafapnoe (OSA) und zur Diagnose und Therapie von Obstruktionen und Vibrationen im Pharynxbereich offenbart, wobei die Vorrichtung intraoral angewendet wird. Die Vorrichtung besteht aus mindestens einer Oraltraverse und aus am hinteren Ende der Oraltraversen angeordneten Mitteln zur Stabilisierung des Velums, wobei die Mittel individuell an die Anatomie des Patienten anpassbar sind, und während der intraoralen Anwendung der Vorrichtung das Velum von der Rachenwand fernhält und das Bindegewebe im oberen Pharynx hinter dem Velum stabilisiert, um Obstruktionen zu verhindern.

Die US 2016/0022473 A1 offenbart ein intraorales Gerät zur Manipulation des Musculus Uvulae und der Uvula. Das Gerät umfasst ein an einer Zahnschiene befestigtes Trägerelement mit einem Mittel zur Regulierung des Musculus uvulae und der Uvula mit Hilfe einer Anpassungsbiegung, welche in eine Nasopharynx eines Benutzers eingreift.

Nachteilig an den zuletzt genannten Vorrichtungen zur Verhinderung des Schnarchens ist, dass diese während des Schlafes getragen werden und lediglich mechanisch eine Vibration des Gaumensegels oder ein Zurückfallen der Zunge verhindern. Diese Vorrichtungen sorgen somit nicht dauerhaft für eine Verringerung des Schnarchens. Ein weiterer Nachteil bei den bekannten Vorrichtungen ist, dass diese nicht ausreichend an die Geometrie des Mundraumes beziehungsweise des Gaumens und des Gaumensegels des Nutzers angepasst werden können, was zu Würgereizen beim Einsetzen führen kann.

Ferner ist nachteilig an den bekannten Vorrichtungen, dass diese den Kieferbewegungen, insbesondere des Unterkiefers, der betroffenen Person nicht ausreichend Rechnung tragen.

### Darstellung der Erfindung: Aufgabe, Lösung, Vorteile

Der vorliegenden Erfindung liegt die Aufgabe zugrunde, eine Vorrichtung zur Schnarchtherapie bereitzustellen, mit welcher eine Stärkung der Gaumenmuskulatur erreicht werden kann, welche lediglich während kurzer Trainingsphasen tagsüber getragen werden muss, welche individuell anpassbar ist und mit welcher zudem die Bewegungen des Unterkiefers des Trägers berücksichtigt werden. Ferner liegt der Erfindung die Aufgabe zugrunde, ein Verfahren zur Herstellung einer Vorrichtung zur Schnarchtherapie und eine Verwendung einer Vorrichtung zur Schnarchtherapie bereitzustellen, mit welcher die vorgenannten Vorteile erzielt werden.

Zur Lösung der der Erfindung zugrundeliegenden Aufgabe wird eine Vorrichtung zur Schnarchtherapie vorgeschlagen, umfassend einen Befestigungsabschnitt zur Befestigung an Zähnen eines Oberkiefers und einen Funktionsabschnitt, wobei der Funktionsabschnitt im am Oberkiefer angeordneten Zustand in einer dorsalen Richtung, insbesondere nach dorsal, über eine A-Linie hinausragt und an einem weichen Gaumen in Ruhelage anliegt, wobei der Funktionsabschnitt ausgebildet ist, bei einem Schluckvorgang eine Gegenkraft auf am Schluckvorgang beteiligte Muskeln, insbesondere auf den Musculus tensor veli palatini und den Musculus levator veli palatini, auszuüben, wobei der Funktionsabschnitt bogenförmig und/oder gewölbt ausgebildet ist, und wobei eine im Wesentlichen senkrecht zur A-Linie verlaufende Mittellinie des Funktionsabschnitts in Bezug auf eine Okklusionsebene in einem Winkel zwischen 5° und 25° in dorsaler Richtung ansteigend verläuft.

Der Funktionsabschnitt ist bevorzugt an dem Befestigungsabschnitt angeordnet oder befestigt und ragt im am Oberkiefer eines Trägers angeordneten Zustand dorsal über die A-Linie, in Richtung des Rachenraums, hinaus. Der Funktionsabschnitt kann einstückig mit dem Befestigungsabschnitt ausgebildet sein. Insbesondere beginnt in dorsaler Richtung gesehen der Funktionsabschnitt im am Oberkiefer angeordneten Zustand am Übergang zwischen dem harten Gaumen und dem weichen Gaumen.

Die A-Linie bezeichnet die Grenze zwischen dem harten Gaumen und dem weichen Gaumen im Mund eines Menschen. Alternativ, wenn auch nicht bevorzugt, kann die A-Linie auch als die, insbesondere gedachte, Linie zwischen den Weisheitszähnen des Oberkiefers eines Menschen definiert werden. Bevorzugt ist die A-Linie jedoch die Grenze zwischen dem harten Gaumen und dem weichen Gaumen.

Die Vorrichtung ist ausgebildet, um in den Mund eines Menschen eingesetzt und mit dem Befestigungsabschnitt an den Zähnen des Oberkiefers befestigt zu werden, sodass die Vorrichtung sicher im Mund oder am Kiefer angeordnet wird. In der Ruhelage des Gaumens, das heißt wenn keine Schluckbewegung stattfindet, liegt der Funktionsabschnitt am weichen Gaumen an. Dabei liegt der Funktionsabschnitt derart in Ruhelage an dem weichen Gaumen an, dass der weiche Gaumen nach oben, insbesondere in cranialer Richtung, leicht angehoben wird.

Wenn der Träger der Vorrichtung einen Schluckvorgang durchführt, erfolgt eine isometrische Kontraktur oder Kontraktion der Gaumenbögen des Gaumens beziehungsweise des Musculus tensor veli palatini und/oder des Musculus levator veli palatini. Der Funktionsabschnitt ist so ausgebildet, dass bei der Kontraktion oder Kontraktur der Gaumenbögen beziehungsweise der vorgenannten Muskeln eine Gegenkraft gegen die Gaumenbögen bzw. diese Muskeln ausgeübt wird. Hierdurch werden die Muskeln bei jedem Schluckvorgang trainiert und erhalten einen erhöhten Ruhetonus. Im Schlaf verhindert der erhöhte Ruhetonus ein Erschlaffen der Muskeln. Dadurch kann das Schnarchen verhindert oder reduziert werden.

Die Okklusionsebene bezeichnet die Ebene, die zwischen den Zähnen des Oberkiefers und des Unterkiefers verläuft, wenn der Kiefer geschlossen ist, das heißt wenn die Zähne des Oberkiefers und des Unterkiefers aneinander anliegen. Bei einer normalen aufrechten Kopfhaltung ist die Okklusionsebene im Wesentlichen horizontal angeordnet. Bezüglich der Okklusionsebene ist der Funktionsabschnitt in einem Winkel zwischen 5° und 25°, bevorzugt zwischen 10° und 20°, besonders bevorzugt zwischen 14° und 16°, ganz besonders bevorzugt von 15°, ausgerichtet und in dorsaler Richtung ansteigend verlaufend angeordnet. Als Referenz für die Bestimmung des Winkels kann dabei eine Mittellinie des Funktionsabschnittes betrachtet werden. Die Mittellinie des Funktionsabschnittes verläuft in etwa senkrecht zu der A-Linie entlang einer Symmetrieachse der Vorrichtung zur Schnarchtherapie. Diese Mittellinie verläuft im am Oberkiefer angeordneten Zustand der Vorrichtung bezüglich der Okklusionsebene in dorsaler Richtung nach oben, sodass der Funktionsabschnitt besonders gut dem dorsalen Verlauf des weichen Gaumens folgt.

Weiter ist besonders vorteilhaft, dass die Vorrichtung zur Schnarchtherapie nicht in der Nacht getragen werden muss, sondern nur zum Training während des Tages eingesetzt und verwendet wird. Das Training erfolgt somit im Wachzustand durch die Schluckbewegungen des Trägers.

Besonders bevorzugt ist vorgesehen, dass die Vorrichtung zur Schnarchtherapie nur für kurze Zeiträume am Tag getragen werden muss. Beispielsweise ist es möglich, dass die Vorrichtung je Tag zwischen 10 und 60 Minuten, bevorzugt zwischen 20 und 40 Minuten, insbesondere bevorzugt etwa 30 Minuten getragen werden muss, um eine messbare Reduktion des nächtlichen Schnarchens herbeizuführen.

Mit weiterem Vorteil kann vorgesehen sein, dass der Zeitraum in ein, zwei, drei oder mehr Zeitintervalle je Tag aufgeteilt wird, wobei die Zeitintervalle entweder gleich oder unterschiedlich lang sind.

Ganz besonders bevorzugt ist vorgesehen, dass die Tragezeit pro Tag zwei Zeitintervalle ä 15 Minuten beträgt.

Ganz besonders vorteilhaft ist die bogenförmige und/oder gewölbte Ausgestaltung des Funktionsabschnittes. Durch die bogenförmige und/oder gewölbte Ausgestaltung wird die Form des Funktionsabschnitts besonders gut an den weichen Gaumen angepasst. Durch die vorteilhafte Anpassung an den weichen Gaumen braucht der Funktionsabschnitt nicht so weit über die A-Linie dorsal hinauszuragen, dass dieser in Kontakt mit dem Gaumenzäpfchen oder Uvula kommt, wodurch sonst ein Würgereiz ausgelöst werden könnte. Eine bogenförmige und/oder gewölbte Ausgestaltung bedeutet dabei, dass der Funktionsabschnitt im am Oberkiefer angeordneten Zustand bei einer Frontalansicht in Richtung oder entlang der Okklusionsebene eine im Wesentlichen nach oben gewölbte oder bogenförmig abgerundete Formgebung aufweist. Die Okklusionsebene bezeichnet die Ebene, die zwischen den Zähnen des Oberkiefers und des Unterkiefers verläuft, wenn der Kiefer geschlossen ist, das heißt wenn die Zähne des Oberkiefers und des Unterkiefers aneinander anliegen. Bei einer normalen aufrechten Kopfhaltung ist die Okklusionsebene im Wesentlichen horizontal angeordnet. Die gewölbte oder bogenförmige Formgebung kann bspw. durch eine als Ausschnitt einer Zylinderfläche ausgebildete Ausbildung des Funktionsabschnittes erhalten werden.

Von besonderem Vorteil ist zudem, dass die Vorrichtung zur Schnarchtherapie zur Befestigung an den Zähnen eines Oberkiefers vorgesehen ist. Besonders bevorzugt ist die Vorrichtung ausgebildet, ausschließlich an dem Oberkiefer oder an den Zähnen des Oberkiefers befestigt zu werden. Eine Behinderung der Bewegung des Unterkiefers findet somit nicht statt.

Mit weiterem Vorteil kann vorgesehen sein, dass der Befestigungsabschnitt zur Befestigung an den Zähnen des Oberkiefers eine Schiene, insbesondere eine Zahnschiene, zur Befestigung an einem Oberkieferzahnkranz ist, wobei die Schiene bevorzugt den gesamten Oberkieferzahnkranz aufnimmt.

Der als Schiene bzw. Zahnschiene ausgebildete Befestigungsabschnitt weist bevorzugt Aufnahmen für die Zähne des Oberkiefers auf, wobei die Aufnahmen insbesondere formkomplementär zu den Zähnen oder Zahnkronen ausgebildet sind. Durch diese Maßnahme wird ein besonders guter Sitz der Vorrichtung zur Schnarchtherapie im Mund des Trägers gewährleistet. Wenn die Schiene den gesamten Oberkieferzahnkranz aufnimmt, wird der Sitz nochmals verbessert.

Der Befestigungsabschnitt kann jedoch auch als Klammer oder Klammern ausgebildet sein, mit welcher/welchen die Vorrichtung an den Zähnen des Oberkiefers durch eine kraft- oder formschlüssige Verbindung arretiert oder befestigt wird.

Ferner kann vorgesehen sein, dass die im Wesentlichen senkrecht zur A-Linie verlaufende Mittellinie des Funktionsabschnitts in Bezug auf eine Okklusionsebene in einem Winkel zwischen 10° und 20°, bevorzugt zwischen 14° und 16°, besonders bevorzugt von 15°, in dorsaler Richtung ansteigend verläuft.

Mit weiterem Vorteil kann vorgesehen sein, dass der Funktionsabschnitt an einem dorsalen Rand eine dorsale Abdämmung aufweist.

Der dorsale Rand bezeichnet dabei den Rand des Funktionsabschnittes im dorsalen, das heißt dem Rachen zugewandten Bereich. Durch die dorsale Abdämmung wird erreicht, dass ein steter Kontakt zwischen dem Funktionsabschnitt und dem weichen Gaumen ermöglicht wird.

Die dorsale Abdämmung kann so ausgebildet sein, dass ein Zwischenraum zwischen dem Funktionsabschnitt und dem weichen Gaumen in dorsaler Richtung abgedichtet wird. Es kann dann durch die dorsale Abdämmung eine Art Saugnapffunktion zwischen Funktionsabschnitt und weichem Gaumen bereitgestellt werden, wobei die dorsale Abdämmung bei einer Bewegung des weichen Gaumens weg von dem Funktionsabschnitt einen Unterdruck zwischen Funktionsabschnitt und weichem Gaumen entstehen lässt, welcher den Funktionsabschnitt am weichen Gaumen festsaugt.

Durch die Sicherstellung des ständigen Kontaktes wird eine bessere Verbindung zwischen dem Funktionsabschnitt und dem weichem Gaumen hergestellt, wodurch der Trainingseffekt verstärkt wird. Darüber hinaus kann der Funktionsabschnitt aufgrund dieses ständigen Kontakts und der verbesserten Trainingswirkung in dorsaler Richtung schmaler gestaltet werden, so dass ein ausreichender Abstand zwischen dem dorsalen Rand des Funktionsabschnitts und dem Gaumenzäpfchen ermöglicht wird, sodass das Auftreten eines Würgereizes vermieden wird.

Ein weiterer Vorteil der dorsalen Abdämmung besteht darin, dass das Entstehen eines Luftstroms zwischen Funktionsabschnitt und weichem Gaumen verhindert wird.

Ferner kann bevorzugt vorgesehen sein, dass die dorsale Abdämmung eine Anformung, insbesondere ein Wulst oder eine Materialverstärkung, ist, wobei die dorsale Abdämmung besonders bevorzugt auf einer Oberseite des Funktionsabschnitts angeordnet ist, wobei die dorsale Abdämmung weiter bevorzugt ausgebildet ist, einen ständigen Kontakt des Funktionsabschnitts mit dem weichen Gaumen herzustellen.

Durch die Ausbildung der dorsalen Abdämmung als Anformung, insbesondere als Wulst oder Materialverstärkung, kann die dorsale Abdämmung ganz besonders einfach ausgebildet werden. Ferner eignet sich ein Wulst oder eine Materialverstärkung oder eine Anformung auch zur besonders guten Anpassung der dorsalen Abdämmung an die Kontur des weichen Gaumens.

Mit besonderem Vorteil ist vorgesehen, dass die dorsale Abdämmung, insbesondere die Anformung, der Wulst oder die Materialverstärkung, eine Breite von 0,5 mm bis 5,0 mm, bevorzugt zwischen 1,0 mm und 4,0 mm, weiter bevorzugt zwischen 2,0 mm und 4,0 mm, insbesondere bevorzugt circa 3,0 mm, aufweist, und/oder dass die dorsale Abdämmung, insbesondere die Anformung, der Wulst oder die Materialverstärkung, eine Höhe von 0,25 mm bis 2,0 mm, bevorzugt von 0,5 mm bis 1,5 mm, weiter bevorzugt von 0,75 mm bis 1,25 mm, insbesondere bevorzugt von circa 1,0 mm, aufweist.

Die vorgeschlagenen Dimensionen von Höhe und Breite der dorsalen Abdämmung sind so gewählt, dass ein verbesserter Tragekomfort erzielt werden kann und gleichzeitig ein optimaler Trainingseffekt gewährleistet wird. Die Breite wird dabei im Wesentlichen entlang der dorsalen Richtung gemessen, die Höhe senkrecht zur Breite und im Wesentlichen senkrecht zu einer Oberfläche des Funktionsabschnitts.

Weiterhin bevorzugt kann vorgesehen sein, dass die dorsale Abdämmung entlang des im Wesentlichen gesamten Verlaufs des dorsalen Randes des Funktionsabschnittes angeordnet ist.

Der dorsale Rand des Funktionsabschnittes verläuft dabei ausgehend von dem Schnittpunkt der A-Linie mit dem Alveolarkamm einer Kieferseite in dorsaler Richtung den Funktionsabschnitt umlaufend bis zum Schnittpunkt der A-Linie mit dem Alveolarkamm der gegenüberliegenden Kieferseite.

Durch diese im Wesentlichen über den gesamten Verlauf des dorsalen Randes ausgebildete dorsale Abdämmung kann der Saugnapfeffekt und damit auch der Trainingseffekt und der Tragekomfort weiter erhöht werden.

Ferner kann vorgesehen sein, dass der Befestigungsabschnitt ein Okklusionsrelief, insbesondere auf einer Unterseite des Befestigungsabschnitts, weiter insbesondere auf einer Unterseite einer Schiene, bevorzugt für Zähne eines Unterkiefers, aufweist.

Das Okklusionsrelief, insbesondere auf der Unterseite des Befestigungsabschnitts, entspricht dabei den Zähnen und den Zahnprofilen des Unterkiefers. Die Zähne des Unterkiefers können während des Tragens der Vorrichtung bei normalen Kau- oder Sprechbewegungen in das Okklusionsrelief eingreifen, ohne dass es zu einem Unterkiefervorschub oder Unterkieferrückschub kommt. Der Tragekomfort wird dadurch deutlich erhöht, und die Akzeptanz des Nutzers, das Training durchzuführen, wird verbessert.

Weiter bevorzugt kann vorgesehen sein, dass die Vorrichtung einen, insbesondere dentalen, Kunststoff und/oder eine dentale Metalllegierung und/oder einen Keramikstoff umfasst, oder dass die Vorrichtung aus einem, insbesondere dentalen, Kunststoff und/oder einer dentalen Metalllegierung und/oder einem Keramikstoff besteht.

Die Verwendung der vorgeschlagenen Materialien ist besonders geeignet für die Vorrichtung zur Schnarchtherapie, da diese medizinisch und biologisch verträglich sind.

Auch kann die Vorrichtung eine Mischung der Materialien umfassen oder aus diesen bestehen.

Bevorzugt ist vorgesehen, dass die Vorrichtung im am Oberkiefer angeordneten Zustand einen harten Gaumen mit einem Gaumenabschnitt abdeckt und/oder dass die Vorrichtung einen Gaumenabschnitt aufweist, wobei die Formgebung des Gaumenabschnitts einer Innenseite eines harten Gaumens entspricht.

Der Gaumenabschnitt beziehungsweise eine Oberseite des Gaumenabschnitts ist dabei der Innenseite des harten Gaumens angepasst, sodass ein sicherer Sitz und erhöhter Tragekomfort gewährleistet wird. Der Gaumenabschnitt kann eine im Wesentlichen konstante Dicke aufweisen, sodass aufgrund der Wölbung des harten Gaumens ein ausreichender Raum für die Bewegung der Zunge im Mund, beispielsweise beim Sprechen, gewährleistet wird. Ferner hat das Vorsehen eines Gaumenabschnitts den Vorteil, dass die Vorrichtung zur Schnarchtherapie mechanisch stabiler wird und länger haltbar ist.

Bevorzugt sind der Funktionsabschnitt und der Gaumenabschnitt entlang einer Verbindungslinie aneinander angeordnet, insbesondere miteinander, weiter bevorzugt einstückig, verbunden. Insbesondere verläuft die Verbindungslinie im am Oberkiefer angeordneten Zustand parallel und/oder entlang und/oder in einer gemeinsamen Ebene mit der A-Linie.

Weiter bevorzugt können der Gaumenabschnitt und der Funktionsabschnitt, insbesondere eine Mittellinie des Gaumenabschnitts und eine Mittellinie des Funktionsabschnitts, an der Verbindungslinie in einem Winkel zueinander stehen. Die Mittellinie des Funktionsabschnittes und die Mittellinie des Gaumenabschnitts verlaufen in etwa senkrecht zu der A-Linie bzw. zur Verbindungslinie entlang einer Symmetrieachse der Vorrichtung zur Schnarchtherapie. Insbesondere verlaufen die Mittellinie des Funktionsabschnittes und die Mittellinie des Gaumenabschnitts in einer Aufsicht auf die Vorrichtung zur Schnarchtherapie mittig, insbesondere in dorsaler Richtung, wobei die Mittellinie des Funktionsabschnittes in der Aufsicht die Fortführung der Mittellinie des Gaumenabschnitts, insbesondere in dorsaler Richtung, ist.

Alternativ kann jedoch auch vorgesehen sein, dass kein Gaumenabschnitt vorhanden ist, sodass die Zunge im eingesetzten Zustand der Vorrichtung zur Schnarchtherapie in direkten Kontakt mit dem harten Gaumen treten kann. In einer Aufsicht beinhaltet die Vorrichtung dann eine zentrale Aussparung, welche von dem Befestigungsabschnitt und dem zwischen den distalen Enden der Zahnbögen verlaufenden Funktionsabschnitt umrandet wird.

Mit weiterem Vorteil kann vorgesehen sein, dass sich die Vorrichtung, insbesondere der Gaumenabschnitt und/oder der Funktionsabschnitt, im am Oberkiefer befestigten Zustand zwischen den Alveolarkämmen beider Kieferseiten distal erstreckt.

Bevorzugt deckt der Funktionsabschnitt im am Oberkiefer befestigten Zustand den weichen Gaumen über die gesamte Breite des weichen Gaumens bis zu den Alveolarkämmen vollständig ab, bzw. liegt über die gesamte Breite des weichen Gaumens bis zu den Alveolarkämmen an dem weichen Gaumen an.

Der Funktionsabschnitt und/oder der Gaumenabschnitt erstrecken sich somit über die gesamte Kieferbreite zwischen den Alveolarkämmen. Hierdurch wird die Stabilität der Vorrichtung erhöht. Zudem erstreckt sich der Funktionsabschnitt in distaler Richtung über einen größeren Bereich des Gaumensegels beziehungsweise der Gaumenbögen, sodass bei dem Schluckvorgang ein noch stärkerer Trainingseffekt und somit eine Verbesserung des Muskeltonus des Musculus tensor veli palatini und/oder des Musculus levator veli palatini erzielt wird.

Besonders vorteilhaft ist vorgesehen, dass der Funktionsabschnitt entlang einer Mittellinie in dorsaler Richtung eine Breite zwischen 0,5 cm und 2,5 cm, bevorzugt zwischen 1,0 cm und 2,0 cm, insbesondere bevorzugt circa 1,5 cm, aufweist.

Die Ausgestaltung des Funktionsabschnittes, insbesondere die bogenförmige und/oder gewölbte Ausgestaltung, erhöht aufgrund der besseren Anlage an den weichen Gaumen und/oder an das Gaumensegel den Trainingseffekt, sodass der Funktionsabschnitt in dorsaler Richtung nicht bis zum Gaumenzäpfchen reichen muss. Entsprechend kann die Breite des Funktionsabschnittes in dorsaler Richtung, das heißt vom Mundeingang zum Rachen, gesehen, wie vorgeschlagen gewählt werden. Die vorgeschlagenen Abmessungen des Funktionsabschnittes bieten einen optimalen Kompromiss zwischen Trainingseffekt und einem erhöhten Tragekomfort für den Träger.

Weiter bevorzugt kann vorgesehen sein, dass auf dem Funktionsabschnitt, insbesondere auf einer Oberseite des Funktionsabschnittes, ein Ausgleichsmittel angeordnet oder anordbar, insbesondere angeformt oder anformbar, ist, wobei das Ausgleichsmittel bevorzugt eine auf den Funktionsabschnitt auftragbare oder aufgetragene Kunststoffmasse ist, wobei die Kunststoffmasse weiter bevorzugt thermisch oder durch elektromagnetische Strahlung aushärtbar oder ausgehärtet ist.

Das Ausgleichsmittel ist besonders bevorzugt eine thermisch oder durch elektromagnetische Strahlen, beispielsweise IR- oder UV-Strahlung, aushärtbare Kunststoffmasse. Wenn sich im Laufe des Trainings, beispielsweise über einen Zeitraum von mehreren Wochen, der Ruhetonus der Muskeln im weichen Gaumen erhöht, kann der Trainingseffekt nachlassen. Aus diesem Grunde ist es besonders vorteilhaft, wenn insbesondere auf die Oberseite des Funktionsabschnitts das als Kunststoffmasse ausgebildete Ausgleichsmittel aufgetragen wird. Dabei kann das Auftragen beispielsweise durch Aufstreichen oder Auflegen oder Anfügen erfolgen. Durch die Ausgleichsmasse wird die bogenförmige und/oder gewölbte Ausgestaltung des Funktionsabschnittes verstärkt, insbesondere kann durch die Ausgleichsmasse der Winkel der Mittelachse des Funktionsabschnitts zu der Okklusionsebene erhöht werden. Hierdurch und durch das zusätzliche Material des Ausgleichsmittels auf dem Funktionsabschnitt wird die Gegenkraft gegen die Gaumenmuskeln bei einer Schluckbewegung erhöht. Es kann somit dieselbe Vorrichtung zur Schnarchtherapie auch bei einem bereits eingetretenen Trainingseffekt und einer vorhanden Verbesserung des Ruhetonus weiter verwendet werden, ohne dass sie ausgetauscht werden muss. Insbesondere lässt sich die Vorrichtung zur Schnarchtherapie einfach und kostengünstig an den bereits erzielten Trainingsfortschritt anpassen.

Die Ausgleichsmasse kann jedoch auch ein thermoplastisches Material und/oder ein selbstpolymerisierendes Material sein. Ferner ist es möglich, dass die Ausgleichsmasse in einem additiven und/oder generativen, insbesondere in einem 3D-Druckverfahren, hergestellt und/oder geformt, bzw. an dem Funktionselement angeformt wird.

Eine weitere Lösung der der Erfindung zugrundeliegenden Aufgabe besteht in der Bereitstellung eines Verfahrens zur Herstellung einer, insbesondere vorbeschriebenen, Vorrichtung zur Schnarchtherapie umfassend die Schritte:
- Erstellen eines physischen oder digitalen anatomischen Modells eines Oberkiefers, beispielsweise durch Scannen oder Abdruck oder Abformung des Oberkiefers, insbesondere umfassend Zahnbogen, harter Gaumen und weicher Gaumen,
- Anfertigung einer Vorrichtung zur Schnarchtherapie unter Benutzung des anatomischen Modells, wobei ein Befestigungsabschnitt und ein bogenförmiger und/oder gewölbter Funktionsabschnitt gebildet werden, wobei der Funktionsabschnitt im am Oberkiefer angeordneten Zustand in einer dorsalen Richtung, insbesondere nach dorsal, über eine A-Linie hinausragt und am weichen Gaumen in Ruhelage anliegt, und wobei eine im Wesentlichen senkrecht zur A-Linie verlaufende Mittellinie des Funktionsabschnitts in Bezug auf eine Okklusionsebene in einem Winkel zwischen 5° und 25° in dorsaler Richtung ansteigend verläuft,
- gegebenenfalls Anpassung der Vorrichtung durch Einsetzen in einen Oberkiefer eines Menschen und Nachbearbeitung zur Verbesserung des Sitzes der Vorrichtung und/oder der Anlage des Funktionsabschnittes am weichen Gaumen.

Die A-Linie bezeichnet die Grenze zwischen dem harten Gaumen und dem weichen Gaumen im Mund eines Menschen. Alternativ, wenn auch nicht nicht bevorzugt, kann die A-Linie auch als die, insbesondere gedachte, Linie zwischen den Weisheitszähnen des Oberkiefers eines Menschen definiert werden. Bevorzugt ist die A-Linie jedoch die Grenze zwischen dem harten Gaumen und dem weichen Gaumen.

Besonders vorteilhaft ist die bogenförmige und/oder gewölbte Ausgestaltung des Funktionsabschnitts. Durch die bogenförmige und/oder gewölbte Ausgestaltung wird die Form des Funktionsabschnitts besonders gut an den weichen Gaumen angepasst.

Durch die Verwendung eines physischen oder digitalen anatomischen Modells und durch die gegebenenfalls vorgenommene Anpassung der Vorrichtung werden ein besonders hoher Tragekomfort und ein verstärkter Trainingseffekt erzielt.

Die Merkmale und Vorteile für die vorbeschriebene Vorrichtung zur Schnarchtherapie können analog auf das Verfahren zur Herstellung einer Vorrichtung zur Schnarchtherapie übertragen werden. Umgekehrt können mit der vorbeschriebenen Vorrichtung zur Schnarchtherapie die durch die Verfahrensschritte des Verfahrens erzielten Vorteile erhalten werden.

Mit Vorteil kann vorgesehen sein, dass der Schritt der Anfertigung einer Vorrichtung zur Schnarchtherapie unter Benutzung eines physischen anatomischen Modells in einem Folientiefziehverfahren oder durch Auftragen eines thermisch oder durch elektromagnetische Strahlung, insbesondere durch UV-Strahlung, aushärtbaren Materials, insbesondere eines Kunststoffes, auf das physische anatomische Modell und thermisches Aushärten oder Strahlungsaushärten des Materials erfolgt.

Die Anfertigung der Vorrichtung zur Schnarchtherapie unter Benutzung eines physischen anatomischen Modells ist ein einfaches und kostengünstiges Verfahren zur Herstellung der Vorrichtung. Insbesondere kann die Vorrichtung vor Ort in einem Dentallabor hergestellt werden.

Weiter bevorzugt kann vorgesehen sein, dass der Schritt der Anfertigung einer Vorrichtung zur Schnarchtherapie unter Benutzung eines digitalen anatomischen Modells und eines additiven oder generativen Verfahrens, insbesondere eines 3D-Druckverfahrens, und/oder eines Fräsverfahrens erfolgt.

Die Anfertigung der Vorrichtung zur Schnarchtherapie unter Benutzung eines digitalen anatomischen Modells und eines additiven oder generativen Verfahrens, insbesondere eines 3D-Druckverfahrens, und/oder eines Fräsverfahrens stellt eine besonders vorteilhafte Methode zur Herstellung einer Vorrichtung zur Schnarchtherapie dar. Insbesondere können die Vorteile des additiven oder generativen Verfahrens genutzt werden, welche die Funktionalität und den Tragekomfort der Vorrichtung verbessern. Beispielsweise ist es möglich, an dafür geeigneten Stellen Hohlräume in der Vorrichtung bereitzustellen. Hierdurch können das Gewicht und der Materialaufwand verringert werden, bei gleichbleibender Stabilität der Vorrichtung. Zudem entfällt die Anfertigung eines physischen anatomischen Modells des Oberkiefers.

Ferner können Durchbrüche oder ähnliches in die Vorrichtung, insbesondere bei Verwendung eines 3D-Druckverfahrens, eingearbeitet werden, wodurch beispielsweise eine bessere Durchlüftung oder Speichelabfuhr möglich wird.

Die Vorrichtung zur Schnarchtherapie kann jedoch auch aus einem thermoplastischen Material und/oder einem selbstpolymerisierenden Material angefertigt werden.

In einem weiteren vorteilhaften Verfahrensschritt kann vorgesehen sein, dass der Schritt der Anfertigung einer Vorrichtung zur Schnarchtherapie den Schritt des Anformens und/oder des Ausbildens und/oder des Anbringens einer dorsalen Abdämmung auf einem dorsalen Rand des Funktionsabschnitts umfasst.

Zudem ist es vorteilhaft, wenn der Schritt des Anpassens der Vorrichtung das Anordnen, Anformen oder Aufbringen eines Ausgleichsmittels umfasst, wobei das Ausgleichsmittel eine Kunststoffmasse ist, wobei das Ausgleichsmittel auf den Funktionsabschnitt aufgetragen wird, wobei die Kunststoffmasse thermisch oder durch elektromagnetische Strahlung ausgehärtet wird.

Durch diese Maßnahmen kann die mit dem Verfahren hergestellte Vorrichtung zur Schnarchtherapie während der Therapie oder auch bei der ersten Anpassung leichter angepasst werden, um den Sitz im Oberkiefer des Trägers zu verbessern. Ferner kann während der Durchführung der Schnarchtherapie ein gleichbleibender Trainingseffekt gewährleistet werden.

Ferner kann vorgesehen sein, dass der Schritt der Anfertigung einer Vorrichtung zur Schnarchtherapie die Erstellung eines Okklusionsreliefs, insbesondere auf einer Unterseite des Befestigungsabschnitts, weiter insbesondere auf einer Unterseite einer Schiene, bevorzugt für Zähne eines Unterkiefers, unter Benutzung des physischen oder digitalen anatomischen Modells des Unterkiefers umfasst.

Mit besonderem Vorteil kann vorgesehen sein, dass der Schritt der Erstellung eines Okklusionsreliefs unter Benutzung eines physischen oder digitalen anatomischen Modells eines Unterkiefers erfolgt, und/oder dass der Schritt der Erstellung eines Okklusionsreliefs durch Befestigen der Vorrichtung mittels des Befestigungsabschnitts an Zähnen eines Oberkiefers eines Menschen und Andrücken der Zähne eines Unterkiefers erfolgt, wobei die Vorrichtung für diesen Schritt formbar ist oder formbar gemacht wird.

Zudem wird die Bereitstellung einer Vorrichtung zur Schnarchtherapie, welche durch ein vorbeschriebenes Verfahren erhältlich ist, vorgeschlagen.

Ferner wird eine Verwendung einer vorbeschriebenen Vorrichtung zur Schnarchtherapie vorgeschlagen.

Besonders vorteilhaft ist, wenn die Vorrichtung zur Schnarchtherapie bei der Verwendung ausschließlich im Wachzustand, insbesondere nicht im Schlaf, verwendet wird, und insbesondere an Zähnen eines Oberkiefers befestigt wird.

Mit weiterem Vorteil kann vorgesehen sein, dass die Verwendung, insbesondere täglich, für einen kurzen Zeitraum zwischen 10 und 60 Minuten, bevorzugt zwischen 20 und 40 Minuten, insbesondere bevorzugt etwa 30 Minuten, erfolgt.

Mit weiterem Vorteil kann vorgesehen sein, dass der Zeitraum in ein, zwei, drei oder mehr Zeitintervalle je Tag aufgeteilt wird, wobei die Zeitintervalle entweder gleich oder unterschiedlich lang sind.

Ganz besonders bevorzugt ist vorgesehen, dass die Tragezeit pro Tag zwei Zeitintervalle ä 15 Minuten beträgt.

Mit besonderem Vorteil kann vorgesehen sein, dass die Verwendung der Vorrichtung zur Schnarchtherapie nicht in der Nacht und/oder nicht im Schlaf erfolgt.

### Kurze Beschreibung der Zeichnungen

Die Erfindung wird nachstehend näher anhand der Figuren erläutert.

Es zeigen
- Fig. 1: eine perspektivische Ansicht einer Vorrichtung zur Schnarchtherapie,
- Fig. 2: eine an Zähnen eines Oberkiefers befestigte Vorrichtung zur Schnarchtherapie,
- Fig. 3: eine Seitenansicht einer Vorrichtung zur Schnarchtherapie,
- Fig. 4: eine Ansicht entgegen einer dorsalen Richtung einer Vorrichtung zur Schnarchtherapie, und
- Fig. 5: eine Aufsicht auf eine Vorrichtung zur Schnarchtherapie.

### Ausführliche Beschreibung der Zeichnungen

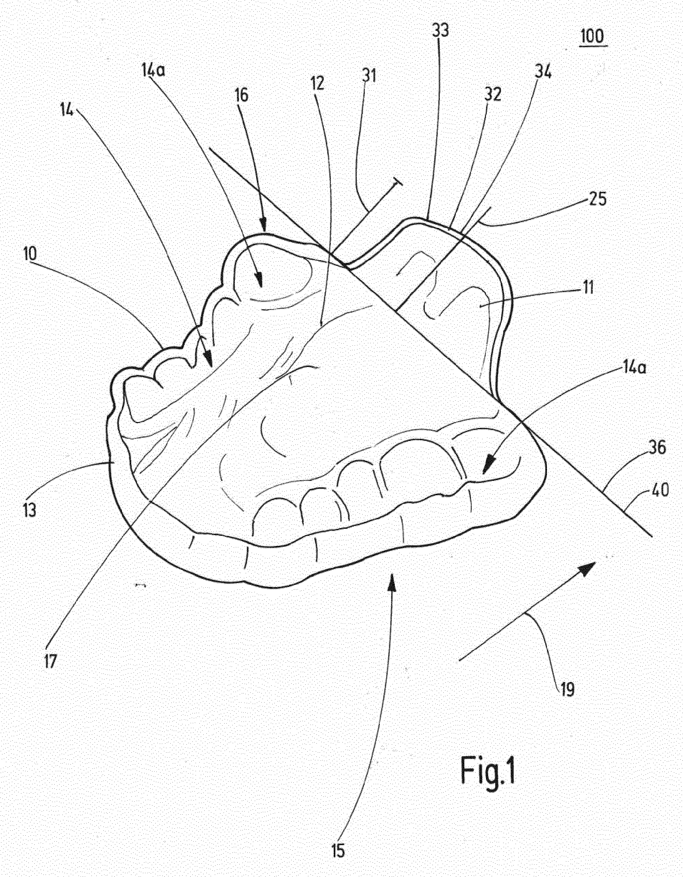

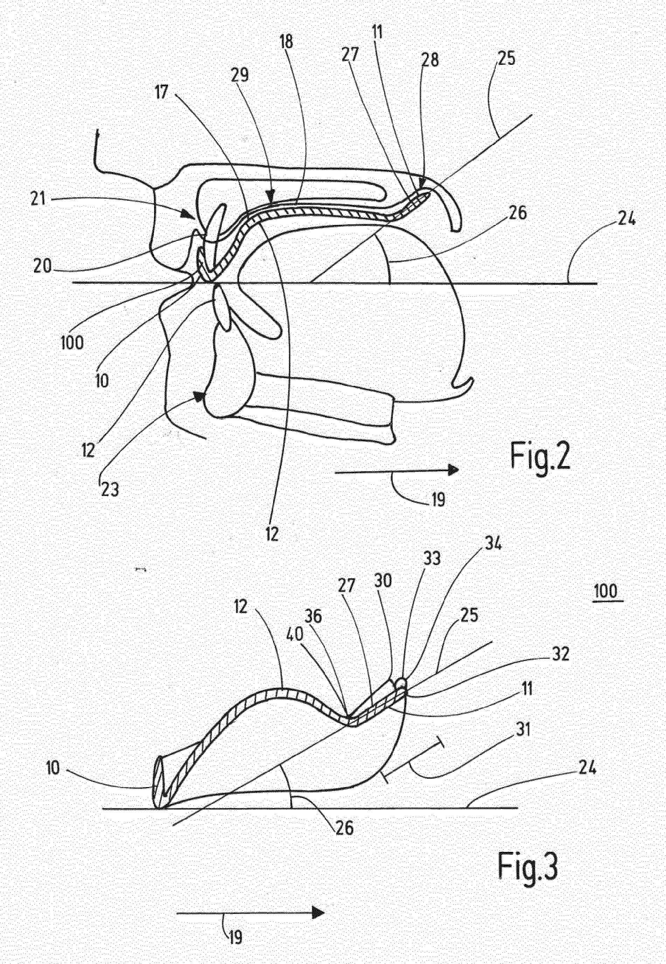

Fig. 1 zeigt eine perspektivische Ansicht von schräg oben auf eine Vorrichtung 100 zur Schnarchtherapie. Fig. 2 zeigt eine Anordnung der Vorrichtung 100 an einem Oberkiefer 21. Die Vorrichtung 100 umfasst einen Befestigungsabschnitt 10, einen Funktionsabschnitt 11 sowie einen Gaumenabschnitt 12. Der Befestigungsabschnitt 10 ist in Form einer Schiene 13 bzw. Zahnschiene ausgebildet, und weist Aufnahmen 14 für die Zähne 20 des Oberkiefers 21 (Fig. 2) eines Menschen auf. Die Aufnahmen 14 sind dabei formkomplementär zu dem Profil der Zähne 20, sodass die Vorrichtung 100 über die Schiene 13 passgenau und sicher auf den Zähnen 20 des Oberkiefers 21 des Menschen befestigt werden kann.

Die sogenannte A-Linie 36, das heißt die Grenze zwischen weichem Gaumen 28 und hartem Gaumen 29 verläuft im am Oberkiefer 21 angeordneten Zustand der Vorrichtung 100 in etwa von den Aufnahmen 14a für die Weisheitszähne auf einer ersten Seite 15 zu den Aufnahmen 14a für die Weisheitszähne auf einer zweiten Seite 16, wobei im gezeigten Fall die A-Linie in dorsaler Richtung hinter den Aufnahmen 14a für die Weisheitszähne verläuft. Die A-Linie 36 ist an der Vorrichtung 100 zur Schnarchtherapie durch eine Verbindungslinie 40 repräsentiert, wobei die Verbindungslinie 40 die Trennlinie zwischen dem Gaumenabschnitt 12 und dem Funktionsabschnitt 11 darstellt. Der Gaumenabschnitt 12 weist eine Gaumenabschnittsoberseite 17 auf, welche im am Oberkiefer 21 befestigten Zustand im Wesentlichen druckfrei, das heißt unter einem sehr geringen Druck oder mit einem leichten Abstand von einer Innenseite 18 des harten Gaumens 29 angeordnet ist. Der Funktionsabschnitt 11 ist in einer dorsalen Richtung 19 hinter dem Befestigungsabschnitt 10 und dem Gaumenabschnitt 12 angeordnet.

Fig. 2 zeigt die Vorrichtung 100 zur Schnarchtherapie im am Oberkiefer 21 angeordneten Zustand im Mund eines Menschen. In der Fig. 2 ist der Kiefer geschlossen dargestellt, sodass die Zähne 20 des Oberkiefers 21 und die Zähne 22 des Unterkiefers 23 in etwa aufeinanderliegen. Zwischen den Zähnen 20, 22 verläuft die sogenannte Okklusionsebene 24. Bezüglich der Okklusionsebene 24 bildet die Mittellinie 25 des Funktionsabschnitts 11 einen Winkel 26. Der Winkel 26 beträgt zwischen 5° und 25° beziehungsweise zwischen 10° und 20°, bevorzugt zwischen 14° und 16°. Wie weiterhin erkennbar ist, liegt eine Oberseite 27 des Funktionsabschnitts 11 an einem weichen Gaumen 28 an. Der Gaumenabschnitt 12 deckt den harten Gaumen 29 ab.

Fig. 3 zeigt einen Schnitt durch die Vorrichtung 100. Der in einem Winkel 26 zur Okklusionsebene 24 verlaufende Funktionsabschnitt 11 ist in dorsaler Richtung 19 hinter dem Gaumenabschnitt 12 angeordnet. Auf der Oberseite 27 des Funktionsabschnittes 11 ist ein Ausgleichsmittel 30 angeordnet. Durch die Anordnung des Ausgleichsmittels 30 auf der Oberseite 27 des Funktionsabschnittes 11 wird der Winkel 26 bezüglich der Okklusionsebene 24 vergrößert Hierdurch wird die Gegenkraft auf die Muskeln des weichen Gaumens 28 bei einer Schluckbewegung erhöht und der Trainingseffekt verstärkt. Das Ausgleichsmittel 30 ist dabei bevorzugt als thermisch oder durch elektromagnetische Strahlung aushärtbarer Kunststoff ausgebildet, welcher auf die Oberseite 27 des Funktionsabschnittes 11 aufgetragen wird und thermisch oder durch elektromagnetische Strahlung ausgehärtet wurde. Die Breite 31 des Funktionsabschnittes 11 gemessen vorzugsweise von der A-Linie 36 bzw. der Verbindungslinie 40 bis zu dem dorsalen Rand 32 des Funktionsabschnitts beträgt zwischen 1 cm und 2 cm, bevorzugt ca. 1,5 cm.

Auf dem dorsalen Rand 32 des Funktionsabschnittes 11 ist eine dorsale Abdämmung 33 angeordnet. Die dorsale Abdämmung 33 ist dabei als Wulst 34 ausgebildet.

Wie besser aus der Fig. 1 erkennbar ist, verläuft die als Wulst 34 ausgebildete dorsale Abdämmung 33 im Wesentlichen entlang des gesamten dorsalen Randes 32 des Funktionsabschnittes 11.

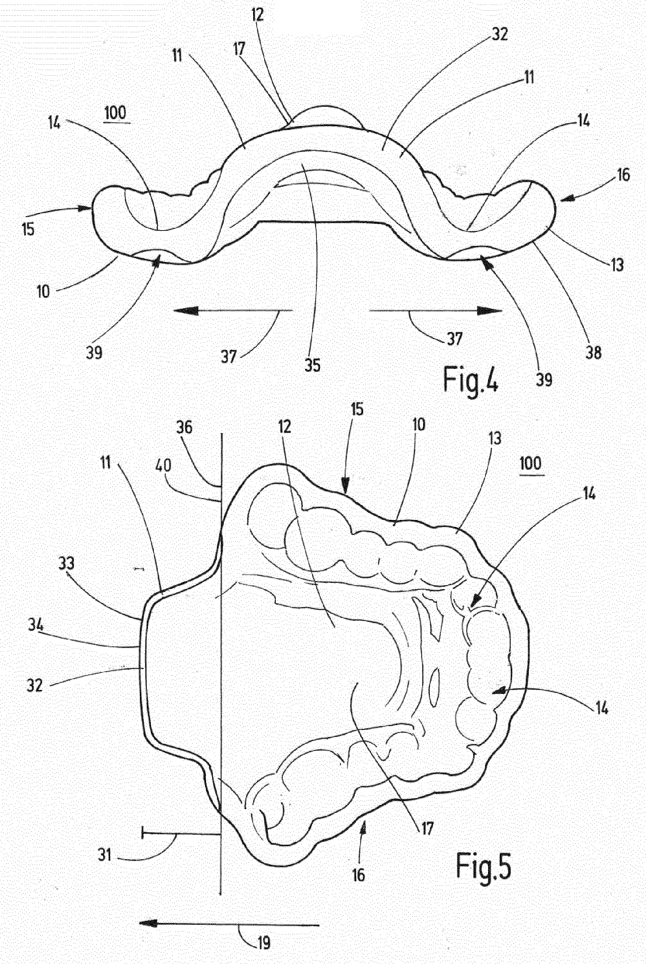

Fig. 4 zeigt eine Aufsicht auf die Vorrichtung 100 entgegen der dorsalen Richtung 19, das heißt im am Oberkiefer 21 angeordneten Zustand aus Richtung des Rachenraums in Richtung der Mundöffnung. Deutlich zu erkennen ist der dorsale Rand 32 des Funktionsabschnittes 11. Der Funktionsabschnitt 11 weist eine bogenförmige Ausgestaltung 35 auf. Der Funktionsabschnitt 11 und der Gaumenabschnitt 12 verlaufen in distalen Richtungen 37 zwischen den Aufnahmen 14 auf der ersten Seite 15 und den Aufnahmen 14 auf der zweiten Seite 16 für die Zähne 20 des Oberkiefers 21. Insbesondere verlaufen somit der Gaumenabschnitt 12 und der Funktionsabschnitt 11 im am Oberkiefer 21 befestigten Zustand zwischen den hier nicht dargestellten Alveolarkämmen beider Kieferseiten. Auf der Unterseite 38 des Befestigungsabschnitts 10 ist ein Okklusionsrelief 39 vorgesehen, in welches die Zähne 22 des Unterkiefers 23 im am Oberkiefer 21 angeordneten Zustand der Vorrichtung 100 eingreifen können.

Fig. 5 zeigt eine Aufsicht auf die Vorrichtung 100 zur Schnarchtherapie. Der Funktionsabschnitt 11 ist in dorsaler Richtung 19 hinter der A-Linie 36 am Befestigungsabschnitt 10 angeordnet.

### Liste der Bezugszeichen

- 100: Vorrichtung
- 10: Befestigungsabschnitt
- 11: Funktionsabschnitt
- 12: Gaumenabschnitt
- 13: Schiene
- 14: Aufnahmen
- 14a: Aufnahme für Weisheitszähne
- 15: Erste Seite
- 16: Zweite Seite
- 17: Gaumenabschnittsoberseite
- 18: Innenseite
- 19: Dorsale Richtung
- 20: Zähne
- 21: Oberkiefer
- 22: Zähne
- 23: Unterkiefer
- 24: Okklusionsebene
- 25: Mittellinie
- 26: Winkel
- 27: Oberseite
- 28: Weicher Gaumen
- 29: Harter Gaumen
- 30: Ausgleichsmittel
- 31: Breite
- 32: Dorsaler Rand
- 33: Dorsale Abdämmung
- 34: Wulst
- 35: Bogenförmige Ausbildung
- 36: A-Linie
- 37: Distale Richtung
- 38: Unterseite
- 39: Okklusionsrelief
- 40: Verbindungslinie

## Patentansprüche

1. Vorrichtung (100) zur Schnarchtherapie, umfassend einen Befestigungsabschnitt (10) zur Befestigung an Zähnen (20) eines Oberkiefers (21) und einen Funktionsabschnitt (11), wobei der Funktionsabschnitt (11) im am Oberkiefer (21) angeordneten Zustand in einer dorsalen Richtung (19) über eine A-Linie hinausragt und an einem weichen Gaumen (28) in Ruhelage anliegt, wobei der Funktionsabschnitt (11) ausgebildet ist, bei einem Schluckvorgang eine Gegenkraft auf am Schluckvorgang beteiligte Muskeln, insbesondere auf den Musculus tensor veli palatini und den Musculus levator veli palatini, auszuüben, wobei der Funktionsabschnitt (11) bogenförmig und/oder gewölbt ausgebildet ist, **dadurch gekennzeichnet, dass** eine im Wesentlichen senkrecht zur A-Linie verlaufende Mittellinie (25) des Funktionsabschnitts (11) in Bezug auf eine Okklusionsebene (24) in einem Winkel (26) zwischen 5° und 25° in dorsaler Richtung (19) ansteigend verläuft, so dass der weiche Gaumen nach oben, insbesondere in cranialer Richtung, leicht angehoben wird.

2. Vorrichtung (100) nach Anspruch 1, **dadurch gekennzeichnet, dass** der Befestigungsabschnitt (10) zur Befestigung an den Zähnen (20) des Oberkiefers (21) eine Schiene (13) zur Befestigung an einem Oberkieferzahnkranz ist, wobei die Schiene (13) bevorzugt den gesamten Oberkieferzahnkranz aufnimmt.

3. Vorrichtung (100) nach Anspruch 1 oder 2, **dadurch gekennzeichnet, dass** die im Wesentlichen senkrecht zur A-Linie verlaufende Mittellinie (25) des Funktionsabschnitts (11) in Bezug auf die Okklusionsebene (24) in einem Winkel (26) zwischen 10° und 20°, bevorzugt zwischen 14° und 16°, besonders bevorzugt von 15°, in dorsaler Richtung (19) ansteigend verläuft.

4. Vorrichtung (100) nach einem der vorgenannten Ansprüche, **dadurch gekennzeichnet, dass** der Funktionsabschnitt (11) an einem dorsalen Rand (32) eine dorsale Abdämmung (33) aufweist, dass bevorzugt die dorsale Abdämmung (33) eine Anformung, insbesondere ein Wulst (34) oder eine Materialverstärkung, ist, wobei die dorsale Abdämmung (33) weiter bevorzugt auf einer Oberseite (27) des Funktionsabschnitts (11) angeordnet ist, wobei die dorsale Abdämmung (33) besonders bevorzugt ausgebildet ist, einen ständigen Kontakt des Funktionsabschnitts (11) mit dem weichen Gaumen (28) herzustellen.

5. Vorrichtung (100) nach Anspruch 4, **dadurch gekennzeichnet, dass** die dorsale Abdämmung (33), insbesondere die Anformung, der Wulst (34) oder die Materialverstärkung, eine Breite von 0,5 mm bis 5,0 mm, bevorzugt zwischen 1,0 mm und 4,0 mm, weiter bevorzugt zwischen 2,0 mm und 4,0 mm, insbesondere bevorzugt circa 3,0 mm, aufweist, und/oder dass die dorsale Abdämmung (33), insbesondere die Anformung, der Wulst (34) oder die Materialverstärkung, eine Höhe von 0,25 mm bis 2,0 mm, bevorzugt von 0,5 mm bis 1,5 mm, weiter bevorzugt von 0,75 mm bis 1,25 mm, insbesondere bevorzugt von circa 1,0 mm, aufweist.

6. Vorrichtung (100) nach einem der vorgenannten Ansprüche, **dadurch gekennzeichnet, dass** der Befestigungsabschnitt (10) ein Okklusionsrelief (39), insbesondere auf einer Unterseite (38) des Befestigungsabschnitts (10), weiter insbesondere auf einer Unterseite (38) einer Schiene (13), bevorzugt für Zähne (22) eines Unterkiefers (23), aufweist.

7. Vorrichtung (100) nach einem der vorgenannten Ansprüche, **dadurch gekennzeichnet, dass** die Vorrichtung im am Oberkiefer (21) angeordneten Zustand einen harten Gaumen (28) mit einem Gaumenabschnitt (12) abdeckt, und/oder dass die Vorrichtung einen Gaumenabschnitt (12) aufweist, wobei die Formgebung des Gaumenabschnitts (12) einer Innenseite (18) eines harten Gaumens (29) entspricht, und/oder dass sich die Vorrichtung, insbesondere der Gaumenabschnitt (12) und/oder der Funktionsabschnitt (11), im am Oberkiefer (21) befestigten Zustand zwischen den Alveolarkämmen beider Kieferseiten distal erstreckt.

8. Vorrichtung (100) nach einem der vorgenannten Ansprüche, **dadurch gekennzeichnet, dass** der Funktionsabschnitt (11) entlang einer Mittellinie (25) in der dorsalen Richtung (19) eine Breite (31) zwischen 0,5 cm und 2,5 cm, bevorzugt zwischen 1,0 cm und 2,0 cm, insbesondere bevorzugt circa 1,5 cm, aufweist.

9. Vorrichtung (100) nach einem der vorgenannten Ansprüche, **dadurch gekennzeichnet, dass** auf dem Funktionsabschnitt (11), insbesondere auf einer Oberseite (27) des Funktionsabschnitts (11), ein Ausgleichsmittel (30) angeordnet oder anordbar, insbesondere angeformt oder anformbar, ist, wobei das Ausgleichsmittel (30) bevorzugt eine auf den Funktionsabschnitt (11) auftragbare oder aufgetragene Kunststoffmasse ist, wobei die Kunststoffmasse weiter bevorzugt thermisch oder durch elektromagnetische Strahlung aushärtbar oder ausgehärtet ist.

10. Verfahren zur Herstellung einer Vorrichtung (100) zur Schnarchtherapie nach einem der Ansprüche 1 bis 9, umfassend die Schritte:
- Erstellen eines physischen oder digitalen anatomischen Modells eines Oberkiefers (21), beispielsweise durch Scannen oder Abdruck oder Abformung des Oberkiefers (21), insbesondere umfassend Zahnbogen, harter Gaumen (29) und weicher Gaumen (28),
- Anfertigung einer Vorrichtung (100) zur Schnarchtherapie unter Benutzung des anatomischen Modells, wobei ein Befestigungsabschnitt (10) und ein bogenförmiger und/oder gewölbter Funktionsabschnitt (11) gebildet werden, wobei der Funktionsabschnitt (11) im am Oberkiefer (21) angeordneten Zustand in einer dorsalen Richtung (19) über eine A-Linie hinausragt und am weichen Gaumen (28) in Ruhelage anliegt, und wobei eine im Wesentlichen senkrecht zur A-Linie verlaufende Mittellinie (25) des Funktionsabschnitts (11) in Bezug auf eine Okklusionsebene (24) in einem Winkel (26) zwischen 5° und 25° in dorsaler Richtung (19) ansteigend verläuft, so dass der weiche Gaumen nach oben, insbesondere in cranialer Richtung, leicht angehoben wird,
- gegebenenfalls Anpassung der Vorrichtung (100) durch Einsetzen in einen Oberkiefer (21) eines Menschen und Nachbearbeitung zur Verbesserung des Sitzes der Vorrichtung (100) und/oder der Anlage des Funktionsabschnitts (11) am weichen Gaumen (28).

11. Verfahren nach Anspruch 10, **dadurch gekennzeichnet, dass** der Schritt der Anfertigung einer Vorrichtung (100) zur Schnarchtherapie unter Benutzung eines physischen anatomischen Modells in einem Folientiefziehverfahren oder durch Auftragen eines thermisch oder durch elektromagnetische Strahlung aushärtbaren Materials, insbesondere eines Kunststoffes, auf das physische anatomische Modell und thermisches Aushärten oder Strahlungsaushärten des Materials erfolgt, und/oder dass der Schritt der Anfertigung einer Vorrichtung (100) zur Schnarchtherapie unter Benutzung eines digitalen anatomischen Modells und eines additiven oder generativen Verfahrens, insbesondere eines 3D-Druckverfahrens, und/oder eines Fräsverfahrens erfolgt.

12. Verfahren nach Anspruch einem der Ansprüche 10 oder 11, **dadurch gekennzeichnet, dass** der Schritt der Anfertigung einer Vorrichtung (100) zur Schnarchtherapie den Schritt des Anformens und/oder des Ausbildens und/oder des Anbringens einer dorsalen Abdämmung (33) auf einem dorsalen Rand (32) des Funktionsabschnitts (11) umfasst, und/oder dass der Schritt des Anpassens der Vorrichtung (100) das Anordnen, Anformen oder Aufbringen eines Ausgleichsmittels (30) umfasst, wobei das Ausgleichsmittel (30) eine Kunststoffmasse ist, wobei das Ausgleichsmittel auf den Funktionsabschnitt (11) aufgetragen wird, wobei die Kunststoffmasse thermisch oder durch elektromagnetische Strahlung ausgehärtet wird, und/oder dass der Schritt der Anfertigung einer Vorrichtung (100) zur Schnarchtherapie die Erstellung eines Okklusionsreliefs (39), insbesondere auf einer Unterseite (38) des Befestigungsabschnitts (10), weiter insbesondere auf einer Unterseite (38) einer Schiene (13), bevorzugt für Zähne (22) eines Unterkiefers (23), unter Benutzung des physischen oder digitalen anatomisches Modells des Unterkiefers (23) umfasst.

## Claims

1. Device (100) for a therapy against snoring, comprising a fastening section (10) for fastening to teeth (20) of an upper jaw (21) and a functional section (11), wherein the functional section (11), when arranged on the upper jaw (21), projects beyond an A-line in a dorsal direction (19) and rests against a soft palate (28) in the rest position, wherein the functional section (11) is configured to counteract in the swallowing process the muscles involved in the swallowing process, in particular the tensor veli palatini muscle and the levator veli palatini muscle, wherein the functional section (11) is formed arched and / or curved, **characterized in that** a centre line (25) of the functional section (11), wherein the centre line (25) runs substantially perpendicular to the A-line, with respect to an occlusal plane (24) inclines at an angle (26) between 5° and 25° in the dorsal direction (19), so that the soft palate is slightly lifted upwards, in particular in the cranial direction.

2. Device (100) according to claim 1, **characterized in that** the fastening section (10) for fastening to the teeth (20) of the upper jaw (21) is a splint (13) for fastening to a maxillary tooth rim, wherein the splint (13) preferably accommodates the entire maxillary tooth rim.

3. Device (100) according to claim 1 or 2, **characterized in that** the centre line (25) of the functional section (11) running substantially perpendicular to the A-line with respect to an occlusal plane (24) inclines at an angle (26) of between 10° and 20°, preferably of between 14° and 16°, particularly preferably of 15°, in the dorsal direction (19).

4. Device (100) according to one of the preceding claims, **characterized in that** the functional section (11) has a dorsal damming (33) on a dorsal edge (32), that preferably the dorsal damming (33) is a moulding, in particular a bulge (34) or a material reinforcement, wherein the dorsal damming (33) is further preferably arranged on an upper side (27) of the functional section (11), wherein the dorsal damming (33) is particularly preferably configured to enable a permanent contact of the functional section (11) with the soft palate (28).

5. Device (100) according to claim 4, **characterized in that** the dorsal damming (33), in particular the moulding, the bulge (34) or the material reinforcement has a width of 0.5 mm to 5.0 mm, preferably between 1.0 mm and 4.0 mm, more preferably between 2.0 mm and 4.0 mm, particularly preferably approximately 3.0 mm, and / or that the dorsal damming (33), in particular the moulding, the bulge (34) or the material reinforcement has a height of 0.25 mm to 2.0 mm, preferably from 0.5 mm to 1.5 mm, more preferably from 0.75 mm to 1.25 mm, particularly preferably of approximately 1.0 mm.

6. Device (100) according to one of the preceding claims, **characterized in that** the fastening section (10) has an occlusion relief (39), in particular on an underside (38) of the fastening section (10), further in particular on an underside (38) of a splint (13), preferably for teeth (22) of a lower jaw (23).

7. Device (100) according to one of the preceding claims, **characterized in that** the device, when arranged on the upper jaw (21), covers a hard palate (29) with a palate section (12), and / or that the device has a palate section (12), wherein the shape of the palate section (12) corresponds to an inside (18) of a hard palate (29), and / or that the device, in particular the palate section (12) and / or the functional section (11), when fastened on the upper jaw (21), extends distally between the alveolar ridges on both sides of the jaw.

8. Device (100) according to one of the preceding claims, **characterized in that** the functional section (11) along a centre line (25) in the dorsal direction (19) has a width (31) between 0.5 cm and 2.5 cm, preferably between 1.0 cm and 2.0 cm, particularly preferably about 1.5 cm.

9. Device (100) according to one of the preceding claims, **characterized in that** a compensating means (30) is arranged or can be arranged, in particular is moulded or can be moulded, on the functional section (11), in particular on an upper side (27) of the functional section (11), wherein the compensating means (30) is preferably a plastic compound that can be applied or is applied to the functional section (11), wherein the plastic compound further preferably can be hardened or is hardened thermally or by means of electromagnetic radiation.

10. Method for producing a device (100) for a therapy against snoring according to one of claims 1 to 9, comprising the following steps:
- preparation of a physical or digital anatomical model of an upper jaw (21), for example by scanning or impression or moulding of the upper jaw (21), in particular comprising dental arch, hard palate (29) and soft palate (28),
- manufacturing of a device (100) for a therapy against snoring using the anatomical model, wherein a fastening section (10) and an arched and / or curved functional section (11) are formed, wherein the functional section (11), when arranged on the upper jaw (21), projects beyond an A-line in a dorsal direction (19) and rests on the soft palate (28) in the rest position, wherein a centre line (25) of the functional section (11), wherein the centre line (25) runs substantially perpendicular to the A-line, with respect to an occlusal plane (24) inclines at an angle (26) between 5° and 25° in the dorsal direction (19), so that the soft palate is slightly lifted upwards, in particular in the cranial direction,
- if necessary, adapting the device (100) by inserting it into an upper jaw (21) of a person and postprocessing to improve the fit of the device (100) and / or the contact of the functional section (11) on the soft palate (28).

11. Method according to claim 10, **characterized in that** the step of manufacturing a device (100) for a therapy against snoring using a physical anatomical model is carried out in a foil thermoforming process or by applying a material which can be hardened thermally or by electromagnetic radiation, in particular a plastic, to the physical anatomical model and thermal hardening or radiation hardening of the material, and / or that the step of manufacturing a device (100) for a therapy against snoring is carried out using a digital anatomical model and an additive or generative method, in particular a 3D printing method, and / or a milling method.

12. Method according to one of claims 10 or 11, **characterized in that** the step of manufacturing a device (100) for a therapy against snoring comprises the step of moulding and / or forming and / or applying a dorsal damming (33) on a dorsal edge (32) of the functional section (11), and / or **in that** the step of adapting the device (100) comprises arranging, moulding or applying a compensating means (30), wherein the compensating means (30) is a plastic compound, wherein the compensating means is applied on the functional section (11), wherein the plastic compound is hardened thermally or by electromagnetic radiation, and / or **in that** the step of manufacturing a device (100) for a therapy against snoring comprises the creation of an occlusion relief (39), in particular on an underside (38) of the fastening section (10), further in particular on an underside (38) of a splint (13), preferably for teeth (22) of a lower jaw (23), using the physical or digital anatomical model of the lower jaw (23).

## Revendications

1. Dispositif (100) pour le traitement contre le ronflement, comprenant un tronçon de fixation (10) pour la fixation sur des dents (20) d'une mâchoire supérieure (21) et un tronçon fonctionnel (11), dans lequel le tronçon fonctionnel (11), dans l'état disposé sur la mâchoire supérieure (21), fait saillie dans une direction dorsale (19) sur une ligne A et repose à l'état de repos sur un palais mou (28), dans lequel le tronçon fonctionnel (11) est conçu, lors d'une déglutition, pour exercer une contre-force sur des muscles impliqués dans la déglutition, en particulier sur le muscle tenseur du voile du palais et le muscle élévateur du voile du palais, dans lequel le tronçon fonctionnel (11) est arqué et/ou incurvé, **caractérisé en ce qu'**une ligne médiane (25) du tronçon fonctionnel (11) essentiellement verticale à la ligne A est ascendante par rapport à un plan d'occlusion (24) dans un angle (26) entre 5° et 25° dans la direction dorsale (19), de sorte que le palais mou est légèrement soulevé vers le haut, en particulier dans la direction crânienne.

2. Dispositif (100) selon la revendication 1, **caractérisé en ce que** le tronçon de fixation (10) pour la fixation sur les dents (20) de la mâchoire supérieure (21) est un rail (13) pour la fixation sur une couronne de dents de la mâchoire supérieure, dans lequel le rail (13) reçoit de préférence la totalité de la couronne de dents de la mâchoire supérieure.

3. Dispositif (100) selon la revendication 1 ou 2, **caractérisé en ce que** la ligne médiane (25) du tronçon fonctionnel (11) essentiellement verticale à la ligne A est ascendante par rapport au plan d'occlusion (24) dans un angle (26) entre 10° et 20°, de préférence entre 14° et 16°, particulièrement de préférence de 15°, dans la direction dorsale (19).

4. Dispositif (100) selon l'une des revendications précédentes, **caractérisé en ce que** le tronçon fonctionnel (11) présente un renflement dorsal (33) sur un bord dorsal (32), que de préférence le renflement dorsal (33) est une formation, en particulier un bourrelet (34) ou un renforcement de matériau, dans lequel le renflement dorsal (33) est disposé de manière plus préférée sur une partie supérieure (27) du tronçon fonctionnel (11), dans lequel le renflement dorsal (33) est conçu de manière particulièrement préférée, pour créer un contact permanent du tronçon fonctionnel (11) avec le palais mou (28).

5. Dispositif (100) selon la revendication 4, **caractérisé en ce que** le renflement dorsal (33), en particulier la formation, le bourrelet (34) ou le renforcement de matériau, présente une largeur de 0,5 mm à 5,0 mm, de préférence entre 1,0 mm et 4,0 mm, plus encore de préférence entre 2,0 mm et 4,0 mm, particulièrement de préférence d'environ 3,0 mm, et/ou que le renflement dorsal (33), en particulier la formation, le bourrelet (34) ou le renforcement de matériau, présente une hauteur de 0,25 mm à 2,0 mm, de préférence de 0,5 mm à 1,5 mm, plus encore de préférence de 0,75 mm à 1,25 mm, particulièrement de préférence d'environ 1,0 mm.

6. Dispositif (100) selon l'une des revendications précédentes, **caractérisé en ce que** le tronçon de fixation (10) présente un relief d'occlusion (39), en particulier sur une face inférieure (38) du tronçon de fixation (10), plus particulièrement sur une face inférieure (38) d'un rail (13), de préférence pour des dents (22) d'une mâchoire inférieure (23).

7. Dispositif (100) selon l'une des revendications précédentes, **caractérisé en ce que** le dispositif, dans l'état disposé sur la mâchoire supérieure (21), recouvre un palais dur (29) par un tronçon palatal (12), et/ou que le dispositif présente un tronçon palatal (12), dans lequel la forme du tronçon palatal (12) correspond à une face intérieure (18) d'un palais dur (29), et/ou que le dispositif, en particulier le tronçon palatal (12) et/ou le tronçon fonctionnel (11), dans l'état disposé sur la mâchoire supérieure (21), s'étend entre les chambres alvéolaires des deux côtés de mâchoire.

8. Dispositif (100) selon l'une des revendications précédentes, **caractérisé en ce que** le tronçon fonctionnel (11) présente une largeur (31) entre 0,5 cm et 2,5 cm, de préférence entre 1 cm et 2 cm, particulièrement de préférence d'environ 1,5 cm, le long d'une ligne médiane (25) dans la direction dorsale (19).

9. Dispositif (100) selon l'une des revendications précédentes, **caractérisé en ce qu'**un moyen de compensation (30) est disposé ou peut être disposé, en particulier formé ou peut être formé, sur le tronçon fonctionnel (11), en particulier sur une face supérieure (27) du tronçon fonctionnel (11), dans lequel le moyen de compensation (30) est de préférence une masse de plastique pouvant être appliquée ou appliquée sur le tronçon fonctionnel (11), dans lequel la masse de plastique est durcissable ou peut être durcie plus encore de préférence de manière thermique ou par rayonnement électromagnétique.

10. Procédé de fabrication d'un dispositif (100) pour le traitement contre le ronflement selon l'une des revendications 1 à 9, comprenant les étapes de :
- création d'un modèle anatomique physique ou numérique d'une mâchoire supérieure (21), par exemple par scannage ou empreinte ou modelage de la mâchoire supérieure (21), comprenant en particulier: arc dentaire, palais dur (29) et palais mou (28),
- fabrication d'un dispositif (100) pour le traitement contre le ronflement en utilisant le modèle anatomique, dans lequel un tronçon de fixation (10) et un tronçon fonctionnel (11), arqué et/ou incurvé sont formés, dans lequel le tronçon fonctionnel (11), dans l'état disposé sur la mâchoire supérieure (21), fait saillie dans une direction dorsale (19) sur une ligne A et repose à l'état de repos sur le palais mou (28), et dans lequel une ligne médiane (25) du tronçon fonctionnel (11) essentiellement verticale à la ligne A est ascendante par rapport à un plan d'occlusion (24) dans un angle (26) entre 5° et 25° dans la direction dorsale (19), de sorte que le palais mou est légèrement soulevé vers le haut, en particulier dans la direction crânienne,
- le cas échéant, adaptation du dispositif (100) par insertion dans une mâchoire supérieure (21) d'un humain et retouche afin d'améliorer le logement du dispositif (100) et/ou l'appui du tronçon fonctionnel (11) sur le palais mou (28).

11. Procédé selon la revendication 10, **caractérisé en ce que** l'étape de fabrication d'un dispositif (100) pour le traitement contre le ronflement est effectuée en utilisant un modèle anatomique physique dans un procédé d'emboutissage profond de feuille ou par application d'un matériau pouvant être durci de manière thermique ou par rayonnement électromagnétique, en particulier d'un plastique, sur le modèle anatomique physique et durcissement thermique ou durcissement par rayonnement du matériau, et/ou que l'étape de fabrication d'un dispositif (100) pour le traitement contre le ronflement est effectuée en utilisant un modèle anatomique numérique et un procédé additif ou génératif, en particulier un procédé d'impression 3D et/ou un procédé de fraisage.

12. Procédé selon l'une des revendications 10 ou 11, **caractérisé en ce que** l'étape de fabrication d'un dispositif (100) pour le traitement contre le ronflement comprend l'étape de modelage et/ou de formation et/ou de mise en place d'un renflement dorsal (33) sur un bord dorsal (32) du tronçon fonctionnel (11) et/ou que l'étape d'adaptation du dispositif (100) comprend l'agencement, le modelage ou l'application d'un moyen de compensation (30), dans lequel le moyen de compensation (30) est une masse de plastique, dans lequel le moyen de compensation est appliqué sur le tronçon fonctionnel (11), dans lequel la masse de plastique est durcie de manière thermique ou par rayonnement électromagnétique, et/ou que l'étape de fabrication d'un dispositif (100) pour le traitement contre le ronflement comprend la fabrication d'un relief d'occlusion (39), en particulier sur une face inférieure (38) du tronçon de fixation (10), plus particulièrement sur une face inférieure (38) d'un rail (13), de préférence pour des dents (22) d'une mâchoire inférieure (23), en utilisant le modèle anatomique physique ou numérique de la mâchoire inférieure (23).
